Europäisches Patentamt

European Patent Office

Office européen des brevets

(11) Publication number: **0 262 948 B1**

# EUROPEAN PATENT SPECIFICATION

(45) Date of publication of patent specification: **15.04.92**  (51) Int. Cl.5: **C07C 59/01**, C07C 51/367

(21) Application number: **87308659.9**

(22) Date of filing: **30.09.87**

(54) **Process for producing omega-hydroxy fatty acids.**

(30) Priority: **01.10.86 JP 231253/86**

(43) Date of publication of application:
**06.04.88 Bulletin 88/14**

(45) Publication of the grant of the patent:
**15.04.92 Bulletin 92/16**

(84) Designated Contracting States:
**CH DE FR GB LI**

(56) References cited:
**GB-A- 316 158**
**US-A- 2 066 533**

**ORGANIC SYNTHESES COLLECTIVE, vol. 4, 1963, pages 635-638; L.J. DURHAM et al.: "Methyl hydrogen hendecanedioate"**

(73) Proprietor: **NIPPON MINING COMPANY LIMIT-ED**
**12-32, Akasaka 1-chome Minato-ku Tokyo(JP)**

(72) Inventor: **Yokota, Tadafumi c/o Nippon Mining Co., Ltd.**
**17-35 Niizominami 3-chome Toda-shi Saitama(JP)**
Inventor: **Okino, Hiroshi c/o Nippon Mining Co., Ltd.**
**17-35 Niizominami 3-chome Toda-shi Saitama(JP)**
Inventor: **Takagi, Motoyoshi c/o Nippon Min-ing Co., Ltd.**
**17-35 Niizominami 3-chome Toda-shi Saitama(JP)**

(74) Representative: **Pearce, Anthony Richmond et al**
**MARKS & CLERK Alpha Tower Suffolk Street Oueensway Birmingham B1 1TT(GB)**

Rank Xerox (UK) Business Services

## Description

This invention relates to a process of producing ω-hydroxy fatty acids. ω-Hydroxy fatty acids are useful as a raw material for synthesis of medicaments and as a raw material for synthesis of macrocyclic lactones which are used for perfumes, etc., and further can widely be used as a raw material for synthesis of various polymers.

## BACKGROUND OF THE INVENTION

As processes of producing ω-hydroxy fatty acids, there have been proposed a process of subjecting ω-hydroxyalkyl-γ-butyrolactones or ω-acyloxyalkyl-γ-butyrolactones to a catalytic reaction in the presence of a hydrocracking catalyst and in the co-presence of a hydrogen gas as described in U.S. Patents 4,244,873 and 4,246,182; a process of converting 13-oxobicyclo[10,4,0]-hexadecene[1(12)] into a lactone and ring-opening the lactone by a Wolff-Kishner method of a Huang-Minlon method as described in Japanese Patent Publication No. 21464/86; a process of reducing and ring-opening tetrahydroundecanoic acid in the presence of a hydrogenation catalyst and a solid acid catalyst as described in U.S. Patent 4,419,292; and a process of synthesizing 15-hydroxypentadecanoic acid from 1,12-dodecanolide via five steps as described in Japanese Patent Publication No. 3093/72.

However, since the above-described methods use complicated compounds as the starting materials, and since expensive raw materials are used, production costs are increased.

US-A-2066533 discloses a process of producing hydroxy acids and lactones by hydrogenation of dibasic acids and esters thereof having more than one carbon atom per carboxyl group, said hydrogenation being catalysed by a hydrogenating metal or its oxide, or a mixture of such metals and/or their oxides (eg copper chromite).

GB-A-316158 discloses a process for the manufacture of ω-hydroxy aliphatic monocarboxylic acids by reduction of monoesters of aliphatic dicarboxylic acids or salts thereof (eg monoethyl oxalate, potassium ethyl oxalate and monomethyl, monoethyl or monopropyl esters of malonic acid or succinic acid) using a hydrogenation catalyst such as nickel, copper, platinum, palladium, cobalt or iron.

In addition, while processes of individually reducing esters or free acids using a copper-chromium oxide catalyst are known (e.g., A. Guyer et al, Helvetica Chimica Acta, Vol. 38, pp. 976-982 (1955)), it has never been reported that a compound having an ester group and a carboxyl group at both terminals of the same molecule can be catalytically reduced. Hence, it has been difficult to predict the reduction product obtained by catalytically reducing such a compound under severe conditions of high temperature and high pressure.

## SUMMARY OF THE INVENTION

An object of this invention is to provide a process for producing an ω-hydroxy fatty acid at low production cost and with good efficiency using inexpensive raw materials by a simplified method.

As the result of extensive investigations for solving the aforesaid problems, it has now been discovered that when a relatively inexpensive, long-chain dicarboxylic acid as a starting material is monoesterified and then reduced in the presence of a copper-chromium oxide catalyst, the ester group is unexpectedly preferentially reduced to provide an ω-hydroxy fatty acid.

Thus this invention relates to a process for producing an ω-hydroxy fatty acid by the step of hydrogenation of a monoester of an aliphatic dicarboxylic acid having from 10 to 18 carbon atoms in the presence of a copper-chromium oxide catalyst.

## DETAILED DESCRIPTION OF THE INVENTION

To obtain lactones which are useful as raw materials for synthesis of medicaments or perfumes, it is preferred to use a monoester of an aliphatic dicarboxylic acid having from preferably 12 to 17 carbon atoms as the aforesaid dicarboxylic acid monoester.

The aliphatic dicarboxylic acid which is used in the present invention may be saturated or unsaturated. Examples of saturated aliphatic dicarboxylic acids include 1,10-decanedioic acid, 1,11-undecanedioic acid, 1,12-dodecanedioic acid, 1,13-tridecanedioic acid, 1,14-tetradecanedioic acid, 1,15-pentadecanedioic acid, 1,16-hexadecanedioic acid, 1,17-heptadecanedioic acid, and 1,18-octadecanedioic acid or 2-methyl or 3-methyl substituted compounds of these acids; and an example of unsaturated aliphatic dicarboxylic acids is 7-hexadecene-1,16-dioic acid.

The monoester which is used in the invention can be easily obtained by esterifying an aliphatic dicarboxylic acid with an alcohol, converting the diester into a monosalt of an alkali metal or alkaline earth metal using an alkali metal or alkaline earth metal hydroxide, and then replacing the metal with an acid (Organic Syntheses, Collective Volume 4, pp. 635-648 (1963)).

Examples of the alkali metal include lithium, sodium, potassium, and rubidium; and examples of the alkaline earth metal include beryllium, magnesium, calcium, strontium, and barium, with barium and calcium being preferred.

It is preferred for easily separating and purifying the product to use a lower alcohol having from 1 to 4 carbon atoms (such as methanol, ethanol, propanol, and butanol) as the alcohol for the aforesaid esterification.

The esterification is carried out by heating a mixture of one part by weight of the aliphatic dicarboxylic acid with from about 1 to 4 parts by weight of the alcohol, from the 0.5 to 3 parts by weight of an organic solvent (e.g., benzene, toluene, or xylene), and from about 0.1 to 0.5 part by weight of a mineral acid (e.g., sulfuric acid or hydrochloric acid) at a temperature of from about 60 to 120°C for from about 3 to 8 hours.

In addition, the aliphaic dicarboxylic acid which is used in the invention can be produced at relatively low production cost by oxidizing an alphatic compound having a corresponding carbon atom number in the presence of a microorganism (U.S. Patents 3,843,466 and 4,275,158).

The copper-chromium oxide catalyst which is used in the invention is one containing copper oxide and chrominum oxide as active components, which is suitably added with barium, manganese, or silica as a promotor. As this catalyst, one having a CuO content of from about 40 to 60% by weight, a $Cr_2O_3$ content of from about 35 to 55% by weight, and a content of the promotor element of from 0 to about 10% by weight is preferred. The catalyst can be, as a matter of course, used in the form of a carrier-supported catalyst having the above-described components supported on a carrier. Also, it is convenient and preferred to use, as the catalyst, a commercially available copper chromite ($CuO \cdot CuCr_2O_4$) catalyst which is used for producing higher alcohols by hydrogenating glycerides (Adkins, Organic Reaction, Vol. 8, pp. 1-27 (1954)).

It is preferred that the hydrogenation in this invention is performed under conditions of a hydrogen pressure of from about 50 to 300 kg/cm$^2$ and a temperature of from about 150 to 350°C, and or achievement of the reaction, any of a suspension bed system, a fluidized bed system, or a fixed bed system can properly be employed. In this case, if the hydrogen pressure is less than about 50 kg/cm$^2$ or the temperature is lower than about 150°C, the hydrogenation rate is insufficient, undesirably leading to a reduction in yield. A higher reaction temperature or hydrogen pressure is better because the reaction rate increases to improve the yield. However, if the reaction temperature exceeds about 350°C, decomposition of the raw material and product occurs, undesirably leading to a reduction in yield. Also, if the hydrogen pressure exceeds about 300 kg/cm$^2$, a remarkable improvement in reaction rate cannot be expected, and a hydrogen pressure higher than about 300 kg/cm$^2$ is not preferred from the viewpoints of economy and safety.

Further, the reaction is preferably carried out under the conditions that a weight hourly space velocity (WHSV) is from about 0.5 to 20 hr$^{-1}$ and that a molar ratio of hydrogen to the aliphatic dicarboxylic acid is from about 10/1 to 1,000/1. Moreover, the raw materials may be provided for the reaction either directly or after being dissolved in an organic solvent such as dioxane, hexane, or diethyl glycol dimethyl ether.

The above-described reaction condition can be properly selected by considering the long-chain dicarboxylic acid monoester as the raw material, the activity of the catalyst, and the kind of solvent used, if any.

An ω-hydroxy fatty acid obtained after the reaction is sometimes in a partially polyesterified state and thus, if desired, it is further hydrolyzed by an alkali. The hydrolysis of the polymer can be carried out by adding to one mol of the polymer from about 2 to 20 mols of the alkali (e.g., potassium hydroxide or sodium hydroxide) as an aqueous solution thereof having a concentration of from about 3 to 10% by weight and heating the mixture at a temperature of from about 80 to 100°C for from about 1 to 5 hours. The hydrolyzed mixture is then subjected to precipitation by acid and extracted with an organic solvent such as diethyl ether.

The ω-hydroxy fatty acid thus obtained can be polymerized by a known method to form a linear polyester which is then depolymerized upon heating in the presence of a depolymerization catalyst, whereby a macrocyclic lactone can be produced (Japanese Patent Publication No. 25033/76).

According to this invention, by the catalytic action of copper-chromium oxides, the ester group of a long-chain dicarboxylic acid monoester is preferentially reduced by hydrogen while leaving a carboxylic group to provide an ω-hydroxy fatty acid.

The present invention will now be illustrated in greater detail by reference to the following Examples, but the present invention is not to be construed as being limited thereto. Unless otherwise indicated, all parts, percents and ratios are by weight.

Example 1

In a 200-milliliter autoclave were placed 5.73 g (20 mmol) of pentadecanedioic acid monomethyl ester, 0.29 g of a commercially available copper-chromium oxide catalyst (CuO 44% by weight, $Cr_2O_3$ 42% by weight, BaO 6.7% by weight, MnO 3.8% by weight; surface area: 40 to 60 $m^2$/g), and 25 mℓ of dioxane, and after sealing therein hydrogen at an initial hydrogen pressure of 120 kg/$cm^2$, the reaction was performed for 2 hours while heating the mixture at 230°C. Then, the solvent was distilled off in vacuo from the reaction mixture, and 50 mℓ of water was added to the residue, followed by heating at 100°C for 0.5 hour. After adding thereto an aqueous solution of potassium hydroxide (2.61 g of KOH/10 mℓ of water), the resulting mixture was heated at 100°C for 2 hours to perform hydrolysis. To the hydrolysis product were added 50 mℓ of diethyl ether and 5 mℓ of concentrated hydrochloric acid, to extract the product into the ether layer. The ether layer was recovered, washed with an aqueous saturated sodium chloride solution, and dehydrated over anhydrous magnesium sulfate. Thereafter, the solvent was distilled off to provide ω-hydroxypentadecanoic acid. The yield determined by gas chromatography was 52.4%.

Example 2

The same procedures as in Example 1 were followed, except that 6.01 g (20 mmol) of pentadecanedioic acid monoethyl ester was used in place of the pentadecanedioic acid monomethyl ester, to obtain ω-hydroxypentadecanoic acid. The yield of the product was 53.1%.

Example 3

The same procedures as in Example 1 were followed, except that 4.89 g (20 mmol) of dodecanedioic acid monomethyl ester was used in place of the pentadecanedioic acid monomethyl ester, to obtain ω-hydroxydodecanoic acid. The yield of the product was 52.9%.

Example 4

The same procedures as in Example 1 were followed, except that 6.29 g (20 mmol) of heptadecanedioic acid monomethyl ester was used in place of the pentadecanedioic acid monomethyl ester, to obtain ω-hydroxyheptadecanoic acid. The yield of the product was 52.6%.

Example 5

The same procedures as in Example 1 were followed, except that the initial hydrogen pressure and the heating temperature were changed as shown in Table 1 below. As a result, ω-hydroxypentadecanoic acid was obtained in yields shown in the same Table.

Table 1

| Hydrogenation Temperature (°C) | Initial Hydrogen Pressure (kg/$cm^1$) | Yield (%) |
|---|---|---|
| 230 | 80 | 40 |
| 230 | 140 | 63 |
| 215 | 120 | 37 |
| 254 | 120 | 54 |

As described above, since in this invention, a long-chain dicarboxylic acid monoester can be reduced by hydrogen in the presence of a copper-chromium oxide catalyst, the invention permits inexpensive raw materials to be used and ω-hydroxy fatty acids to be produced by a simplified method, at low production cost, and also with good efficiency.

While the invention has been described in detail and with reference to specific embodiments thereof, it will be apparent to one skilled in the art that various changes and modifications can be made therein without departing from the spirit and scope thereof.

**Claims**

4

EP 0 262 948 B1

1. A process for producing an ω-hydroxy fatty acid comprising the step of hydrogenation of a monoester of an aliphatic dicarboxylic acid having from 10 to 18 carbon atoms in the presence of a copper-chromium oxide catalyst.

2. A process as claimed in claim 1, wherein the dicarboxylic acid monoester is a monoester of an aliphatic dicarboxylic acid having from 12 to 17 carbon atoms.

3. A process as claimed in claim 1, further comprising the steps of (a) reacting an aliphatic dicarboxylic acid having from 10 to 18 carbon atoms and an alcohol to provide an ester; (b) hydrolyzing said ester with an alkali metal or alkaline earth metal hydroxide to provide a monosalt of an alkali metal or alkaline earth metal; and then (c) reacting said monosalt with an acid to obtain the dicarboxylic acid monoester.

4. A process as claimed in claim 3, wherein the alkaline earth metal hydroxide is barium hydroxide or calcium hydroxide.

5. A process as claimed in claim 3 or 4, wherein said alcohol contains from 1 to 4 carbon atoms.

6. A process as claimed in any preceding claim, wherein said copper-chromium oxide catalyst comprises at least one of barium, manganese and silica.

7. A process as claimed in any one of claims 1 to 5, wherein said copper-chromium oxide catalyst is a copper-chromite catalyst.

8. A process as claimed in any preceding claim, wherein said hydrogenation is performed at a hydrogen pressure of from about 50 to 300 kg/cm$^2$ and a temperature of from about 150° to 350°C.

9. A process as claimed in any one of claims 1 to 5, wherein said copper-chromium oxide catalyst contains from about 40 to 60% by weight of CuO, from about 35 to 55% by weight of $Cr_2O_3$, and from 0 to about 10% by weight of a promotor element.

10. A process as claimed in claim 1 or 2, wherein the product of said hydrogenation step comprises a polyester and said process comprises the further step of hydrolysing said polyester with an alkali to obtain said ω-hydroxy fatty acid.

11. A process for producing a macrocyclic lactone comprising the steps of:
    (a) hydrogenating a monoester of an aliphatic dicarboxylic acid having from 10 to 18 carbon atoms in the presence of a copper-chromium oxide catalyst to obtain an ω-hydroxy fatty acid;
    (b) polymerising said ω-hydroxy fatty acid to form a linear polyester; and
    (c) heating said polyester in the presence of a depolymerisation catalyst to produce a macrocyclic lactone.

**Revendications**

1. Un procédé pour produire un ω-hydroxy-acide gras comprenant l'étape d'hydrogénation d'un monoester d'un acide dicarboxylique aliphatique en $C_{10}$-$C_{18}$ en présence d'un catalyseur à l'oxyde de cuivre-chrome.

2. Un procédé selon la revendication 1, dans lequel le monoester d'acide dicarboxylique est un monoester d'un acide dicarboxylique aliphatique en $C_{12}$-$C_{17}$.

3. Un procédé selon la revendication 1, comprenant en outre les étapes suivantes :
   (a) on fait réagir un acide dicarboxylique aliphatique en $C_{10}$-$C_{18}$ et un alcool pour donner un ester ;
   (b) on hydrolyse ledit ester par un hydroxyde de métal alcalin ou alino-terreux pour donner un monosel de métal alcalin ou alcalino-terreux ; et ensuite,
   (c) on fait réagir ledit monosel avec un acide pour obtenir le monoester d'acide dicarboxylique.

4. Un procédé selon la revendication 3, dans lequel ledit hydroxyde de métal alcalino-terreux est

5

l'hydroxyde de baryum ou l'hydroxyde de calcium.

5. Un procédé selon la revendication 3 ou 4, dans lequel ledit alcool contient de 1 à 4 atomes de carbone.

6. Un procédé selon l'une quelconque des revendications précédentes dans lequel ledit catalyseur à l'oxyde de cuivre-chrome comprend au moins un composant choisi parmi le baryum, le manganèse et la silice.

7. Un procédé selon l'une quelconque des revendications 1 à 5, dans lequel ledit catalyseur à l'oxyde de cuivre-chrome est un catalyseur au chromite de cuivre.

8. Un procédé selon l'une quelconque des revendications précédentes, dans lequel ladite hydrogénation est effectuée sous une pression d'hydrogène d'environ 50 à 300 kg/cm$^2$ et à une température d'environ 150 à 350°C.

9. Un procédé selon l'une quelconque des revendications 1 à 5, dans lequel ledit catalyseur à l'oxyde de cuivre-chrome contient d'environ 40 à 60 % en poids de CuO, d'environ 35 à 55 % en poids de $Cr_2O_3$ et de 0 à environ 10 % en poids d'un élément promoteur.

10. Un procédé selon la revendication 1 ou 2, dans lequel le produit de ladite étape d'hydrogénation comprend un polyester et ledit procédé comprend l'étape supplémentaire d'hydrolyse dudit polyester par un alcali pour obtenir ledit $\omega$-hydroxy-acide gras.

11. Un procédé pour produire une lactone macrocyclique comprenant les étapes suivantes :
   (a) on hydrogène un monoester d'un acide dicarboxylique aliphatique en $C_{10}$-$C_{18}$ en présence d'un catalyseur à l'oxyde de cuivre-chrome pour obtenir un $\omega$-hydroxy-acide gras ;
   (b) on polymérise ledit $\omega$-hydroxy-acide gras pour former un polyester linéaire ; et
   (c) on chauffe ledit polyester en présence d'un catalyseur de dépolymérisation pour produire une lactone macrocyclique.

**Patentansprüche**

1. Verfahren zum Herstellen einer omega-Hydroxyfettsäure umfassend den Schritt der Hydrierung eines Monoesters einer aliphatischen Dicarbonsäure mit 10 bis 18 Kohlenstoffatomen in Anwesenheit eines Kupfer-Chromoxidkatalysators.

2. Verfahren nach Anspruch 1,
   wobei der Dicarbonsäuremonoester ein Monoester einer aliphatischen Dicarbonsäure mit 12 bis 17 Kohlenstoffatomen ist.

3. Verfahren nach Anspruch 1,
   das weiterhin die Schritte umfaßt:
   (a) Umsetzen einer aliphatischen Dicarbonsäure mit 10 bis 18 Kohlenstoffatomen mit einem Alkohol unter Bildung eines Esters;
   (b) Hydrolysieren des Esters mit einem Alkalihydroxid oder Erdalkalihydroxid unter Bildung eines Monosalzes, eines Alkalimetalls oder Erdalkalimetalls; und anschließend
   (c) Umsetzen des Monosalzes mit einer Säure unter Erhalt des Dicarbonsäuremonoesters.

4. Verfahren nach Anspruch 3,
   wobei das Erdalkalihydroxyd Bariumhydroxid oder Calziumhydroxid ist.

5. Verfahren nach Anspruch 3 oder 4,
   wobei der Alkohol 1 bis 4 Kohlenstoffe enthält.

6. Verfahren nach einem der vorhergehenden Ansprüche,
   wobei der Kupfer-Chromoxidkatalysator mindestens eine Komponente aus Barium, Mangan und Siliziumdioxid umfaßt.

7. Verfahren nach einem der Ansprüche 1 bis 5,
   wobei der Kupfer-Chromoxidkatalysator ein Kupfer-Chromitkatalysator ist.

8. Verfahren nach einem der vorhergehenden Ansprüche,
   wobei die Hydrierung bei einem Wasserstoffdruck von ungefähr 50 bis 300 kg/cm$^2$ und bei einer Temperatur von ungefähr 150°C bis 350°C durchgeführt wird.

9. Verfahren nach einem der Ansprüche 1 bis 5,
   wobei der Kupfer-Chromoxidkatalysator ungefähr 40 bis 60 Gew.% CuO, ungefähr 35 bis 55 Gew.% Cr$_2$O$_3$ und 0 bis ungefähr 10 Gew.% eines Promotorelements enthält.

10. Verfahren nach Anspruch 1 oder 2,
    wobei das Produkt der Hydrierungsstufe einen Polyester umfaßt und das Verfahren den zusätzlichen Schritt des Hydrolysierens des Polyesters mit einem Alkali unter Erhalt der omega-Hydroxyfettsäure umfaßt.

11. Verfahren zum Herstellen eines macrozyklischen Lactons umfassend die Schritte:
    (a) Hydrieren eines Monoesters einer aliphatischen Dicarbonsäure mit 10 bis 18 Kohlenstoffatomen in Anwesenheit eines Kupfer-Chromoxid-Katalysators unter Erhalt einer omega-Hydroxyfettsäure;
    (b) Polymerisieren der omega-Hydroxyfettsäure unter Bildung eines linearen Polyesters und
    (c) Erhitzen des Polyesters in Anwesenheit eines Depolymerisationskatalysators unter Erhalt eines makrozyklisches Lactons.